# EUROPEAN PATENT APPLICATION

(11) **EP 2 746 188 A1**
(43) Date of publication of application: **25.06.2014**
(21) Application number: 12197895.1
(22) Date of filing: 18.12.2012
(51) Int. Cl.: B65D 83/04, A61M 5/00, B65D 5/50, B65D 85/42

(54) **Packaging for containers filled with a pharmaceutical product and having a tubular shaped body**

(71) Applicant: Sanofi-Aventis Deutschland GmbH, 65929 Frankfurt am Main (DE)
(72) Inventor: Kuehn, Bernd, 60323 FRANKFURT AM MAIN (DE)
(74) Representative: Santarelli

(57) **Abstract**

The invention provides a packaging comprising a plurality of containers (10) and a continuous support strip (9) having a plurality of sections (25), each being configured to receive a plurality of said containers successively disposed next to each other with a first constant pitch, and said support strip is configured so that, between first and second successive sections, a jump (26) is formed between first and second containers respectively disposed on said first and second sections, said first and second containers being adjacent and spaced from one another by a second pitch distinct from the first constant pitch provided on said first section which precedes said jump, said first constant pitch and said second pitch being determined according to a predetermined diameter of the containers so that each container of said first section is adjacent to at least one container of said second section.

## Description

### FIELD OF THE INVENTION

The invention relates to a packaging for containers filled with a pharmaceutical product and having a tubular shaped body such as vials, in particular ampoule vials or barrel-type ampoule or syringe barrel.

### BACKGROUND ART

It is well known that ampoules are made from glass or plastic material and filled for instance with a liquid pharmaceutical product.

The ampoules generally comprise a body and a head, the head having a pierceable membrane at its end or being of the self-breakable type and having a pre-breaking line at a junction between the head and the body.

The ampoules are prone to breaking or scratching and therefore have to be suitably protected for instance for transport and/or storage.

It is known packaging for ampoules, comprising a thermoformed receiving member to provide cavities for ampoules and a covering film member made from aluminum and fixed on the receiving member to cover the cavities. The covering film member is removable to extract said ampoules from the cavities. This packaging is similar to a blister for solid pharmaceutical product such as tablets.

It is also known packaging for ampoules, comprising a base member which is plane and made from cardboard and a receiving member also made from cardboard and bonded on the base member. The receiving member comprises a first folded portion and a second folded portion, which are opposite and similar and situated near two opposite ends of the base member. The first and second portions protrude from said base member in a first direction. The first and second portions have a plurality of windows providing spaces to each receive a portion of the body of an ampoule. The receiving member further comprises a third folded portion provided between the first and second folded portions and having a first row of openings and a second row of openings and a portion of material interposed between said first and second rows of openings. The third portion protrudes from the base member in the same first direction. Each opening of the first row, respectively of the second row, faces a window of the first folded portion, respectively of the second folded portion, and is configured to receive the head of the ampoule whose portion of the body is received in this window.

The European patent EP 0 042 336 describes a packaging taking the form of a serie of self-breakable ampoules which are each held tightly in a sheath. Each sheath is formed by two superposed sheets of thermally retractable material which are mutually assembled by welding lines extending on either side of each ampoule. Perforated lines are provided on the sheath between two ampoules so that in order to use an ampoule, a portion of the sheath is detached and the ends of the ampoule are broken without extracting the ampoule of the portion of the sheath.

The European patent EP 0 028 995 describes a packaging for pharmaceutical ampoules cut from a single blank of cardboard and having two foldable portions, each being formed of two hollow parallel lateral supports of rectangular section which are connected together by a flat portion. Each lateral support is provided with openings for receiving the end of the ampoules. The two foldable portions are connected and articulated to each other by a hinge portion.

These above mentioned packaging are generally inserted in a packaging support which forms a box having a rectangular shape. A plurality of these packaging are inserted adjacent to each other or superimposed in the box.

It is also known packaging supports which form dispensers of pharmaceutical tablets. Such dispensers are described for instance in US patents US 2,771,214 and US 4,274,550, in European patent EP 1 794 063 and in UK patent application GB 2 223 001.

These dispensers generally comprise a flexible sheath, like the sheath described in the European patent EP 0 042 336, which comprises housings for tablets. The flexible sheath is rolled up and comprises more or fewer turns and thus the tablets are more or less tight, so that the packaging formed by the sheath and the tablets are more or less compact.

The invention is directed to a packaging for containers filled with a pharmaceutical product and having a tubular shaped body, which is simple, compact and economic.

### SUMMARY OF THE INVENTION

The invention accordingly provides a packaging for containers each filled with a pharmaceutical product and each having a tubular shaped body provided with a predetermined diameter, comprising a plurality of said containers and a receiving member which is made from a flexible material and configured to receive said containers successively disposed near one another, wherein said receiving member is formed by a continuous support strip having a plurality of sections, each section being configured to receive a plurality of said containers successively disposed next to each other with a first constant pitch on said section, and said support strip being configured so that, between two successive sections, respectively a first section and a second section, a jump is formed between a first container and a second container which are respectively disposed on said first and second sections, said first and second containers being adjacent and spaced from each other by a second pitch distinct from the first constant pitch provided on said first section which precedes said jump, said first constant pitch and said second pitch being determined according to said predetermined diameter of said containers so that each container of said first section is adjacent to at least one container of said second section, by virtue of which said containers are arranged to have a compact overall packaging.

The packaging according to the invention thus requires less volume than the packaging mentioned in the background art, for the same density. This means that in the same volume, the density of containers in the packaging according to the invention is greater than the density of pharmaceutical products in the current packaging.

The packaging according to the invention is thus particularly convenient to save packaging volume and/or to increase the density of containers.

The containers which are adjacent and disposed on successive sections are arranged so that they are at least partially nested in order to fill a minimal volume.

The packaging according to the invention is thus particularly simple, compact and economic.

According to features preferred as being very simple, convenient and economical for embodying the packaging according to the invention:
- said support strip is stacked in a zigzag configuration so that all the sections of said support strip are superimposed and each jump corresponds to a folded portion of said support strip between two superimposed sections;
- all the sections of said support strip have a same predetermined number of said containers successively disposed next to each other with the same first constant pitch on said sections;
- said second pitch is greater than said first constant pitch;
- said second pitch is constant;
- said support strip is rolled up in a plurality of turns so that all the sections of said support strip are incurved;
- each section extends along one turn and each turn comprises a jump;
- each section of said support strip has a distinct predetermined number of said containers successively disposed next to each other;
- each section of said support strip has a distinct first constant pitch between said containers successively disposed next to each other on said sections;
- said support strip forms a spiral which has an inner end and an outer end, and said first constant pitch on said sections decrease from the section in the vicinity of said inner end to the section at the outer end;
- said second pitch between first and second sections in the vicinity of the inner end is smaller than said first constant pitch of said first section in the vicinity of the inner end; and said second pitch between first and second sections at the outer end is greater than said first constant pitch of said first section at the outer end;
- said support strip is made from a longitudinal single sheet of cardboard;
- said container is a syringe barrel or an ampoule vial having a cylindrical shaped body, which is made from glass or plastic material;
- said packaging further comprises a box having an internal space which is configured to receive said support strip, said support strip being stored in said box; and/or
- said box comprises lateral walls and a window provided in one of them, said window being configured to be through by said support strip in order to dispense said containers.

### BRIEF DESCRIPTION OF THE DRAWINGS

The description of the invention now continues with a detailed description of a preferred embodiment given hereinafter by way of non-limiting example and with reference to the appended drawings. In these drawings:
- Figure 1 is a schematic perspective view of a packaging according to the invention, having a packaging support partially cutaway to show a support strip having containers, the support strip being stacked in zigzag configuration;
- Figures 2 and 3 are respectively schematic top and bottom views of a section of the support strip of Figure 1 in a plane configuration;
- Figure 4 is a schematic front view, taken isolated, of the support strip of Figure 1 having containers and being stacked in zigzag configuration;
- Figure 5 is a schematic top view of sections of the support strip of Figure 1, without container;
- Figure 6 is a perspective view showing a section taken isolated according to a variant of the support strip;
- Figures 7 and 8 are perspective and front views of sections of the support strip of Figure 6, without container;
- Figure 9 is a similar view to Figure 1, according to a variant of the packaging where the support strip is rolled up;
- Figure 10 is a schematic front view, taken isolated, of the support strip of Figure 9, having containers and being rolled up; and
- Figure 11 is a schematic top view of sections of the support strip of Figure 9, without container.

### DETAILED DESCRPTION OF PREFERRED EMBODIMENTS

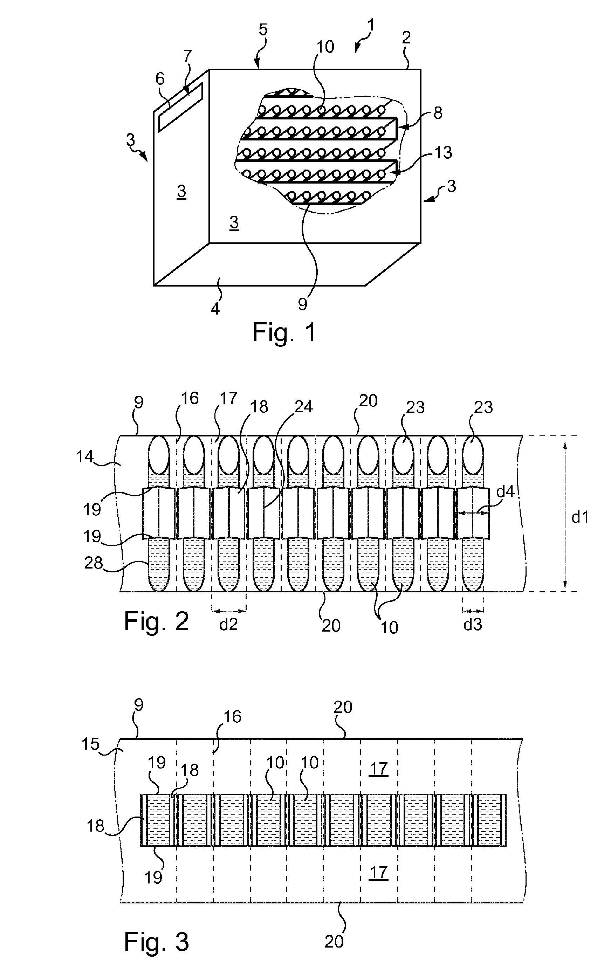

Figure 1 shows a packaging 1 comprising a box 2, also named packaging support, a support strip 9, also named receiving member, and a plurality of pharmaceutical ampoules 10, also named containers, the ampoules 10 being borne by the support strip 9 and stored in an internal space 8 of the box 2 in a predetermined configuration.

The box 2 is here made from cardboard and has a rectangular shape and comprises four lateral walls 3 providing a belt, a bottom wall 4 linked to said four lateral walls 3 and a cover wall 5 linked and articulated to one of said lateral wall 3 by a hinge, in order to form the internal space 8.

The cover wall 5 has an opened position for introducing the support strip 9 and the ampoules 10 in the predetermined configuration and a closed position.

The box 2 further comprises a window 6 formed in one of said lateral wall 3 (here the same lateral wall 3 as that to which the cover wall 5 is linked), said window 6 being configured to create a passage 7 for extracting the support strip 9 and thus for dispensing the ampoules 10. The passage 7 is thus configured to be through by the support strip 9.

The ampoules 10 are here made from glass and are filled with a liquid pharmaceutical product.

Each ampoule 10 has a tubular shaped body 28 which is here cylindrical and provided with a predetermined diameter d3 and a predetermined length. The predetermined diameter d3 of the ampoule 10 is here for instance equal to 8mm and the predetermined length is here for instance equal to 63mm, so that the ampoule 10 has a volume here equal to about 1,7mL.

Each ampoule 10 comprises a head 23 having here a pierceable membrane at its end in order to empty the ampoule 10 (Figure 2).

The support strip 9 is made from a longitudinal single sheet of material, here in cardboard, in order to be flexible (or foldable) and is configured to receive the ampoules 10. The single sheet of cardboard has a thickness here for instance equal to 0,1 mm.

As shown on Figure 1, the support strip 9 is here stacked in a zigzag configuration.

The support strip 9 has here a rectangular shape provided with a length and a width, represented d1, which is smaller than the length. The width d1 of the support strip 9 is here for instance equal to 62mm.

The width of the support strip 9 is defined between two lateral edges 20.

The support strip 9 has an upper face 14 (Figure 2) and a lower face 15 (Figure 3) which is opposite to the upper face 14.

The support strip 9 comprises a plurality of folding lines 16 provided in the width of the support strip 9 between the lateral edges 20.

Two successive folding lines 16 are spaced by a predetermined distance d2, here for instance equal to 20mm.

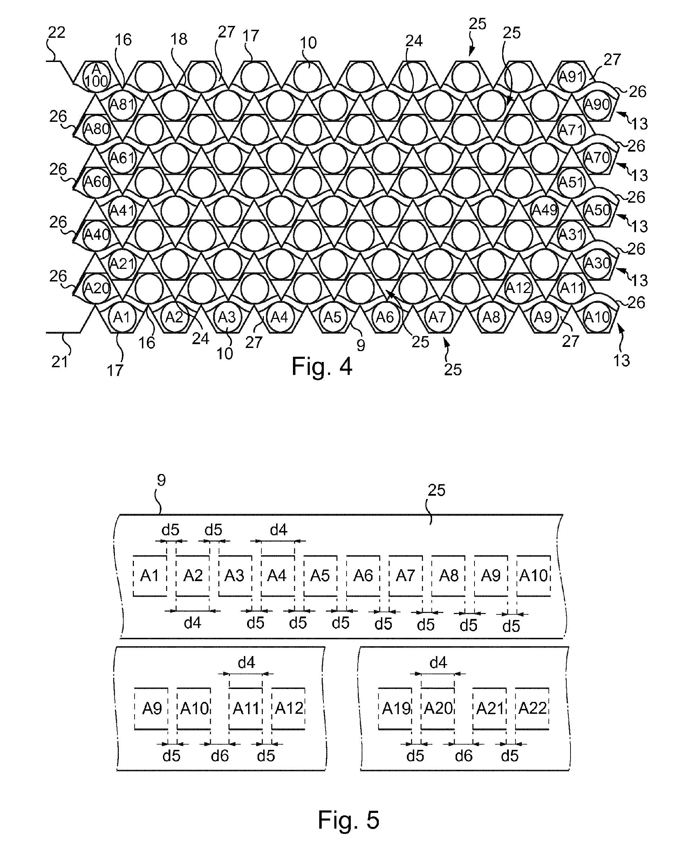

The support strip 9 has a base portion 17 defined between each couple of two successive folding lines 16, except at an inner 21 end and outer end 22 of the support strip 9 (Figure 4).

The support strip 9 further comprises on each base portion 17 two cuts 19 which are parallel and centrally provided on the single sheet of cardboard so that the two cuts 19 define a foldable portion 18 interposed between two parts of the base portion 17.

The two cuts 19 face one another and have a same length so that the foldable portion 18 is provided in the width of the support strip 9 and with a predetermined length d4 which is greater than the diameter d3 of the ampoules 10 and smaller than the distance d2 provided between each couple of successive folding lines 16.

The predetermined length d4 of the foldable portion 18 is here for instance equal to 16mm.

Each couple comprising a base portion 17 and a foldable portion 18 forms a housing 27 (Figure 4) configured to fittingly receive one ampoule 10 according to its predetermined diameter d3.

In other words, as mentioned above, the length d4 of the two cuts 19 is determined in relation to the predetermined diameter d3 of the ampoule 10.

The housing 27 formed by both the base portion 17 and the foldable portion 18 is further configured so that the base portion 17 radially extends in a first direction whereas the foldable portion 18 radially extends in a second direction which is opposite to the first direction when the ampoule 10 is fittingly received in the housing 27.

Each foldable portion 18 comprises a central fold 24 so that each foldable portion 18 is incurved when the ampoule 10 is fittingly received in the housing 27.

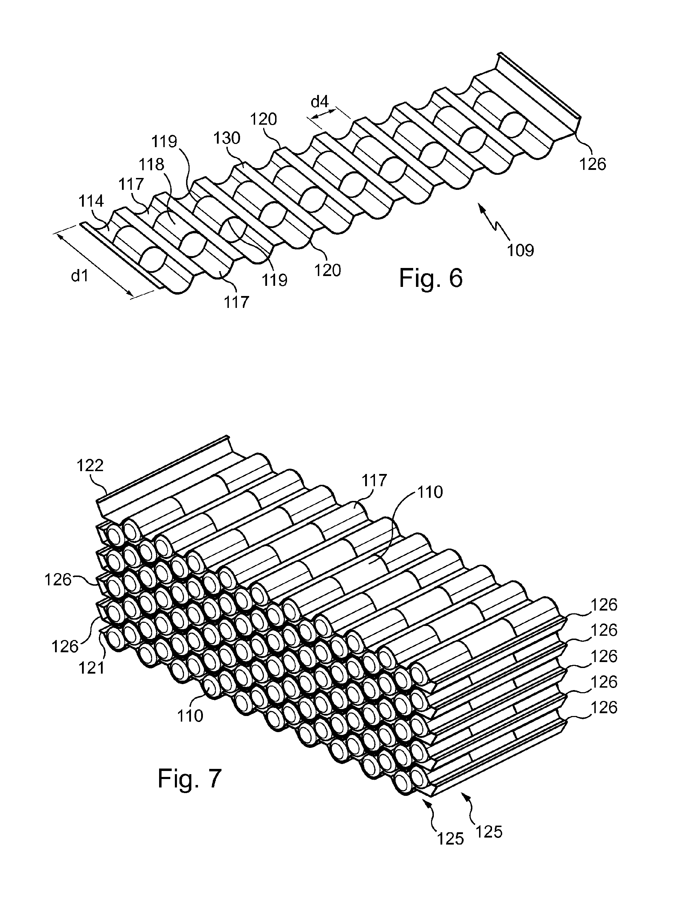

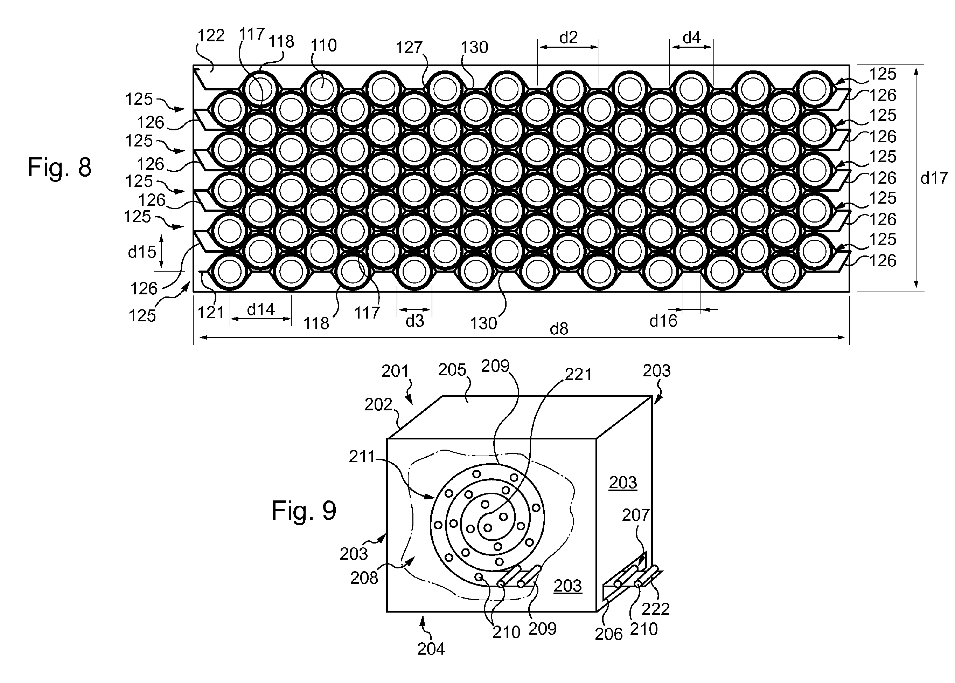

The support strip 9 in Figure 4 is schematically shown and in practice, the two parts of each base portion 17 are also incurved when the ampoule 10 is fittingly received in the housing 27 (see Figures 6 and 8 showing a variant of the support strip).

Each couple comprising a base portion 17 and a foldable portion 18 is thus configured to clamp an ampoule 10, said ampoule 10 having two end portions in contact with the two parts of the base portion 17 and a central portion in contact with the foldable portion 18.

As shown on Figures 1 to 3, the support strip 9 comprises a plurality of housings 27 configured next to each other, each housing being configured in the width of said support strip 9.

We will now describe in detail the zigzag configuration of the support strip 9.

As shown on Figure 4, the support strip 9 forms a continuous receiving member having a plurality of sections 25, each section 25 being configured to receive a plurality of ampoules 10 successively disposed next to each other with a first constant pitch d5 on each section 25. Here, the support strip 9 comprises one hundred housings 27 in each of which an ampoule 10 is fittingly received.

The ampoules 10 are here named A1, A2, A3 ... A100.

The inner end 21 of the support strip 9 is configured to be disposed in the internal space 8 of the box 2, in vicinity of the bottom wall 4 whereas the outer end 22 is configured to be disposed in the internal space 8 of the box 2, in the vicinity of the cover wall 5 and more precisely, the outer end 22 faces the window 6 of the box 2.

The sections 25 of the support strip 9 are superimposed in the zigzag configuration so that one section is in a first position where the base portions 17 of the housings 27 of this section 25 radially extend in a first direction toward the bottom wall 4 of the box 2 and the foldable portions 18 of said housings 27 radially extend in a second direction toward the cover wall 5 of the box 2; and the successive section 25 which is immediately above is in a second position where the base portions 17 of the housings 27 of this section 25 radially extend in a first direction toward the cover wall 5 and the foldable portions 18 of the housings 27 of the section 25 radially extend in a second direction toward the bottom wall 4.

The support strip 9 is further configured so that between two successive sections 25, respectively a first section and a second section, a jump 26 is formed between the two ampoules 10 (for instance A10 and A11 or A40 and A41) which are respectively disposed on the first and second sections.

Each jump 26 corresponds to a folded portion of the support strip 9 between two superimposed sections 25, the folded portions having a length which defines a second pitch d6 distinct from the first constant pitch d5.

The two containers 10 of said first and second sections are thus spaced from each other by this second pitch d6.

The first constant pitch d5 and the second pitch d6 are determined in relation to the predetermined diameter d3 of the ampoules 10.

Here, the first constant pitch d5 is for instance equal to 4mm and the second pitch d6 is for instance equal to 9mm.

The support strip 9 is configured so that every section 25 has a same predetermined number of ampoules 10 successively disposed next to each other with the same first constant pitch d5 on all the sections 25.

Here, the jumps 26 have the same length so that the second pitch d6 is constant and also greater than the first constant pitch d5.

The support strip 9 is thus configured so that each ampoule 10 of one section 25 is adjacent to at least one ampoule 10 of another immediately superimposed section 25.

Figure 4 shows that the ampoules 10 of one section 25 are nested with the ampoules 10 of another immediately superimposed section 25, by virtue of which the ampoules 10 are arranged to have a compact overall packaging 1.

It will be noted that the box 2 in which are introduced the sections 25 has a height here for instance equal to 7cm, a width here for instance equal to 18cm and a depth here for instance equal to 6,3cm, so that the volume of the box 2 is here equal to about 794cm³ for one hundred ampoules 10 in the box 2.

A method for assembling the packaging 1 comprises the step of providing a plurality of containers 10 and the support strip 9 in a flank state.

The method further comprises the step of acting on the support strip 9 to move it from the flank state to a packaging state, packaging state in which the base portion 17 radially extends in a first direction and the foldable portion 18 radially extends in a second direction which is opposite to the first direction.

The method further comprises the step of fittingly inserting the containers 10 into the housings 27.

It will be noted the housings 27 are formed at the same time and the containers 10 are introduced at the same time into the housings 27.

Next, the method comprises the step of stacking up in zigzag configuration the support strip 9 which fittingly bears the container 10 and the step of storing the stacked support strip 9 in the box 2.

Figures 6 to 8 show a variant of the support strip 9 of Figures 2 to 5.

The support strip 109 of Figures 6 to 8 is almost identical to the support strip 9 except that the support strip 109 comprises an intermediate portion 130 between two successive base portions 117 (or two successive housings defined by two parts of a base portion 117 and a foldable portion 118) and the jumps 126 are similar but not identical to the jumps 26. The jumps 126 have each a gutter shape.

The support strip 109 comprises an upper face 114 on which are provided two cuts 119 which define the base portion 117 and the foldable portion 118 between the two lateral edges 120.

The foldable portion 118 and the base portion 117 are configured to form a housing having a width d4 as shown on Figure 6 which is greater than the diameter d3 of the ampoules 10

The ampoules 110 are identical to the ampoules 10 so that d3 is here for instance equal to 8mm and the predetermined width d4 of the housing is here for instance equal to 10mm.

Each intermediate portion 130 has a width d16 (Figure 8) which is here for instance equal to 4,3mm. The width d16 approximately corresponds to the half of the predetermined diameter d3 of the ampoules 110. The width d16 corresponds to the first constant pitch d16 between two successive ampoules 110 on a section 125.

The support strip 109 in Figure 6 is shown as if the ampoules 110 were in the housings, the two parts of each base portion 117 and the foldable portion 118 being incurved.

Figures 7 and 8 show a plurality of sections 125 superimposed in a zigzag configuration.

The ampoules 110 are spaced from each other on the same section 125 by a distance d14 which is here for instance equal to 15,2mm

Two successive jumps 126 are spaced from each other on the same side of the support strip 109 arranged in sections 125 in zigzag configuration by a distance d15 which is here for instance equal to 15mm.

The sections 125 of the support strip 109 are superimposed in the zigzag configuration so that one section is in a first position where the base portions 117 of the housings of this section 125 radially extend in a first direction toward the bottom wall of the box and the foldable portions 118 of said housings radially extend in a second direction toward the cover wall of the box; and the successive section 125 which is immediately above is in a second position where the base portions 117 of the housings of this section 125 radially extend in a first direction toward the cover wall and the foldable portions 118 of the housings of the section 125 radially extend in a second direction toward the bottom wall.

The support strip 109 is further configured so that between two successive sections 125, respectively a first section and a second section, a jump 126 is formed between the two ampoules 110 which are respectively disposed on the first and second sections.

Each jump 126 corresponds to a folded portion of the support strip 109 between two superimposed sections 125, the folded portions having a length which defines a second pitch distinct from the first constant pitch d16.

The two containers 10 of said first and second sections are thus spaced from each other by this second pitch, which is here for instance equal to 15mm.

The first constant pitch d16 and the second pitch are determined in relation to the predetermined diameter d3 of the ampoules 110.

The support strip 109 is configured so that every section 125 has a same predetermined number of ampoules 110 successively disposed next to each other with the same first constant pitch d16 on every section 125.

Here, the jumps 126 have the same length so that the second pitch is constant and also greater than the first constant pitch d16.

The support strip 109 is thus configured so that each ampoule 110 of one section 125 is adjacent to at least one ampoule 110 of another immediately superimposed section 125.

Figures 7 and 8 show that the ampoules 110 of one section 125 are nested with the ampoules 110 of another immediately superimposed section 125, by virtue of which the ampoules 110 are arranged to have a compact overall packaging.

It will be noted that the arrangement shown on Figure 8 has an external height d17 here for instance equal to 5,6cm, a length d8 here for instance equal to 16,2cm and a depth here for instance equal to 6,3cm, so that the volume of the arrangement (which corresponds to the volume of the box in which the arrangement is introduced) is here equal to about 572cm³ for one hundred ampoules 110.

A method for assembling the packaging comprises the step of providing a plurality of containers 110 and the support strip 109 in a flank state.

The method further comprises the step of acting on the support strip 109 to move it from the flank state to a packaging state, packaging state in which the base portion 117 radially extends in a first direction and the foldable portion 118 radially extends in a second direction which is opposite to the first direction.

The method further comprises the step of fittingly inserting the containers 110 into the housings.

It will be noted the housings are formed at the same time and the containers 110 are introduced at the same time into the housings.

Next, the method comprises the step of stacking up in zigzag configuration the support strip 9 which fittingly bears the container 110 and the step of storing the stacked support strip 109 in the box.

Figure 9 shows a variant of the packaging 1 of Figure 1.

The packaging 201 on Figure 9 comprises a box 202, a support strip 209 and a plurality of ampoules 210 which are borne by the support strip 209 and stored in the internal space 208 of the box 202 in a predetermined configuration distinct from the zigzag configuration shown on Figures 1, 4, 7 and 8.

The box 202 is similar to the box 2 and comprises lateral walls 203, a bottom wall 204 and a cover wall 205.

The box 202 further comprises a window 206 formed in one of said lateral walls 203, said window 206 being configured to create a passage 207 for extracting the support strip 209 and thus for dispensing the ampoules 210.

The ampoules 210 are identical to the ampoules 10.

The windows 206 of the box 202 are provided on a lateral wall 203 in the vicinity of the bottom wall 204 whereas the window 6 of the packaging 1 is provided on a lateral wall 3 in the vicinity of the cover wall 5.

The support strip 209 and the ampoules 210 are in a spiral configuration 211.

The spiral 211 comprises a plurality of turns 212 and has an inner end 221 and outer end 222 which is here arranged outside the box 202 after having throughout the passage 207.

The support strip 209 is similar to the support strip 9 shown on Figures 2 and 3 and comprises a plurality of housings each defined by a base portion and a foldable portion, said foldable portion being formed by two cuts provided in the single sheet of cardboard.

We will now describe in detail the spiral configuration 211 of the support strip 209 and the ampoules 210, in reference to Figures 7 and 8.

The support strip 209 is a continuous receiving member having a plurality of sections 225 configured so that each section 225 forms a turn 212 of the spiral 211.

The support strip 209 has the same width d1 as the support strip 9, here for instance equal to 62mm.

The spiral 211 has a predetermined diameter d7 which is here for instance equal to 10cm.

Each section 225 is configured to receive a plurality of ampoules 10 successively disposed next to each other.

Each section 225 has a distinct predetermined number of ampoules 212 successively disposed next to each other so that each section 225 of the support strip 209 has a distinct first constant pitch d10, d12 or d13 between the ampoules 210 successively disposed next to each other on the sections 225.

Each section 225 extends along one turn 212 and thus, each section 225 is incurved and each turn 212 comprises a jump 226.

The ampoule 210 named A1 is not considered as forming a section like the sections 225 because this ampoule is alone at the inner end 221 of the spiral 211.

The support strip 209 is configured so that, between two successive sections 225, respectively a first section and a second section, the jump 226 represents the space between a first and a second ampoules 210 which are respectively disposed on the first and second sections (for instance the ampoules named A7 and A8, A21 and A22, A42 and A43 and A68 and A69).

These first and second ampoules 210 are adjacent and spaced from each other by the second pitch d11 which is distinct from the distinct first constant pitch d10, d12 and d13 provided on the first section 225 which precedes each jump 226.

Here, the distinct first constant pitch d10, d12 and d13 on the respective sections 225 decrease from the section 225 in the vicinity of the inner end 221 to the section 225 at the outer end 222.

Here, the first constant pitch d10 is for instance equal to 4mm, the first constant pitch d12 is for instance equal to 2mm and the first constant pitch d13 is for instance equal to 1mm, whereas the second pitch is for instance equal to 3mm.

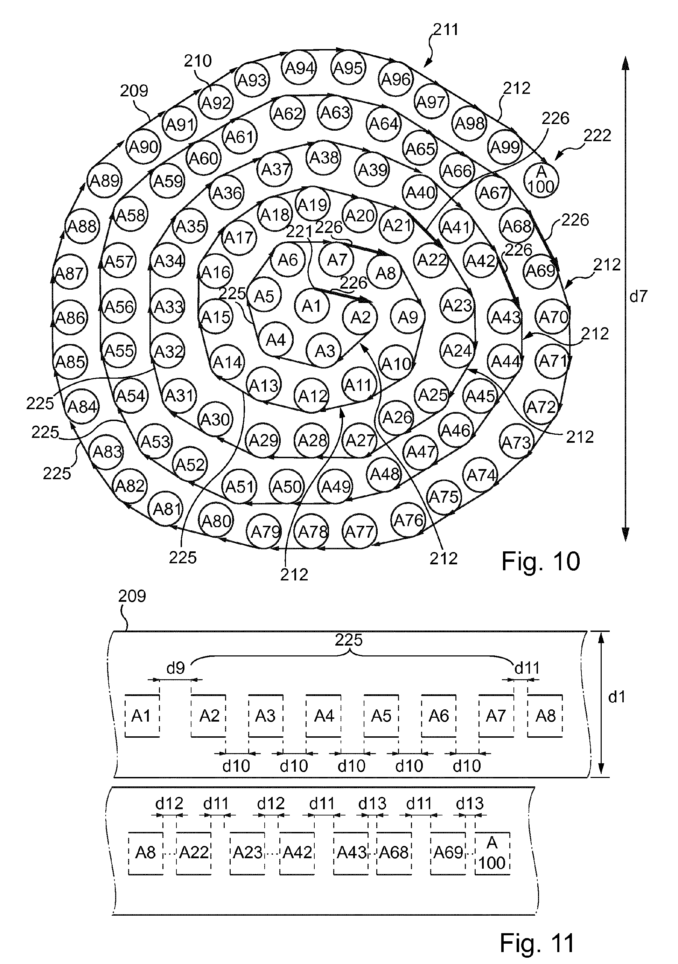

As shown on Figure 10, the ampoule 210 named A1 does not form a section as mentioned above, a jump 226 is created between this ampoule and the successive ampoule 210 named A2. This jump 226 defines a second pitch d9, which is here for instance equal to 7mm.

The ampoules 210 named A2 to A7 on a section 225 are successively disposed next to each other with a first constant pitch d10 which is smaller than the second pitch d9.

The jumps 226 which are then formed on the spiral 211 between respective first and second sections 225 define a similar second pitch d11 which is both smaller than d9 and d10; while the sections 225 which follow the section 225 comprising ampoules 210 named A2 to A7, comprise more and more ampoules 210 because the respective turns 212 have a length which increase and the ampoules 210 of each following sections 225 are successively disposed next to each other with a distinct first constant pitch d12 and d13.

In practice, the ampoules named A8 to A22 and A23 to A42 are spaced two by two with the first constant pitch d12 which is smaller than the first constant pitch d10 and also smaller than the second pitch d11; and the ampoules 210 named A43 to A68 and A69 to A100 are spaced two by two with a first constant pitch d13 which is smaller than the first constant pitch d12.

In the spiral 211, the second pitch d11 between first and second sections 225 in the vicinity of the inner end 221 is smaller than the first constant pitch d10 of the first section 225 in the vicinity of the inner end 221; and the second pitch d 11 between first and second sections 225 at the outer end 222 is greater than the first constant pitch d13 of the first section 225 at the outer end 222.

As shown on Figure 10, the first constant pitch d10, d12 and d13 and the second pitch d11 are determined according to the predetermined diameter of the ampoules 210 so that each ampoule 210 of a first section is adjacent to at least one ampoule 210 of a second section (which is successive).

Thus, the ampoules 210 of a first section 225 are at least partially nested with adjacent ampoules 210 of a second section 225 which follows the first section, by virtue of which the ampoules 210 are arranged to have a compact overall packaging 201.

It will be noted that the box 202 in which are introduced the sections 225 has a height here for instance equal to 11 cm, a width here for instance equal to 11 cm and a depth here for instance equal to 6,3cm, so that the volume of the box 202 is here equal to about 762cm³ for one hundred ampoules 210.

A method for assembling the packaging 201 comprises the step of providing a plurality of containers 210 and the support strip 209 in a flank state.

The method further comprises the step of acting on the support strip 209 to move it from the flank state to a packaging state, packaging state in which the base portion 217 radially extends in a first direction and the foldable portion 218 radially extends in a second direction which is opposite to the first direction.

The method further comprises the step of fittingly inserting the containers 210 into the housings.

It will be noted the housings are formed at the same time and the containers 210 are introduced at the same time into the housings.

Next, the method comprises the step of rolling up the support strip 209 which fittingly bears the containers 210 and the step of storing the rolled support strip 209 in the box 202.

In variants that are not illustrated:
- the support strip is made from a sheet of material and the containers are glued on this sheet;
- the support strip is made from a sheet of material and comprises a receiving element configured to receive containers by click-fit engagement;
- the support strip is not made from a single sheet of material but comprises a plurality of layers, for instance a first layer forming a base portion and a second layer forming a foldable portion which defines housings configured to received containers;
- the foldable portions of the support strip do not comprise a central fold but rather two lateral folds;
- the support strip comprises more than two cuts in order to define more than one foldable portion by housing, for instance, the support strip comprises three or four cuts to form two or three foldable portions by housing;
- the support strip is not made from a single sheet of cardboard but rather of reinforced paper, or laminated paper, or plastic foil material, or another material which is sufficiently tear-resistant but at least partially flexible;
- the support strip comprises a plurality of perforated lines provided in the width of the support strip between the lateral edges;
- the containers are not ampoules as described above but rather syringe barrels;
- the containers are not made from glass but rather from plastic material; and/or
- the head of the ampoules has not a pierceable membrane at its end but rather a pre-breaking line at a junction between the head and the body so that the ampoules are of the self-breakable type.

It should be noted more generally that the invention is not limited to the examples described and represented.

## Claims

1. Packaging for containers (10; 110; 210) each filled with a pharmaceutical product and each having a tubular shaped body (28) provided with a predetermined diameter, comprising a plurality of said containers (10; 110; 210) and a receiving member which is made from a flexible material and configured to receive said containers (10; 110; 210) successively disposed near one another, wherein said receiving member is formed by a continuous support strip (9; 109; 209) having a plurality of sections (25; 125; 225), each section (25; 125; 225) being configured to receive a plurality of said containers (10; 110; 210) successively disposed next to each other with a first constant pitch (d5; d10; d12; d13; d16) on said section (25; 125; 225), and said support strip (9; 109; 209) being configured so that, between two successive sections (25; 125; 225), respectively a first section and a second section, a jump (26; 126; 226) is formed between a first container and a second container which are respectively disposed on said first and second sections, said first and second containers being adjacent and spaced from each other by a second pitch (d6; d11) distinct from the first constant pitch (d5; d10; d12; d13; d16) provided on said first section which precedes said jump (26; 126; 226), said first constant pitch (d5; d10; d12; d13; d16) and said second pitch (d6; d11) being determined according to said predetermined diameter of said containers (10; 110; 210) so that each container (10; 110; 210) of said first section is adjacent to at least one container (10; 110; 210) of said second section, by virtue of which said containers (10; 110; 210) are arranged to have a compact overall packaging (1; 201).

2. Packaging according to claim 1, wherein said support strip (9; 109) is stacked in a zigzag configuration so that all the sections (25; 125) of said support strip (9; 109) are superimposed and each jump (26; 126) corresponds to a folded portion of said support strip (9; 109) between two superimposed sections (25; 125).

3. Packaging according to claim 2, wherein all the sections (25; 125) of said support strip (9; 109) have a same predetermined number of said containers (10; 110) successively disposed next to each other with the same first constant pitch (d5; d16) on said sections (25; 125).

4. Packaging according to one of claims 2 and 3, wherein said second pitch (d6) is greater than said first constant pitch (d5; d16).

5. Packaging according to any one of claims 1 to 4, wherein said second pitch (d6) is constant.

6. Packaging according to claim 1, wherein said support strip (209) is rolled up in a plurality of turns (212) so that all the sections (225) of said support strip (209) are incurved.

7. Packaging according to claim 6, wherein each section (225) extends along one turn (212) and each turn (212) comprises a jump (226).

8. Packaging according to one of claims 6 and 7, wherein each section (225) of said support strip (209) has a distinct predetermined number of said containers (210) successively disposed next to each other.

9. Packaging according to claim 8, wherein each section (225) of said support strip (209) has a distinct first constant pitch (d10; d12; d13) between said containers (210) successively disposed next to each other on said sections (225).

10. Packaging according to claim 9, wherein said support strip (209) forms a spiral which has an inner end (221) and an outer end (222), and said first constant pitch (d10; d12; d13) on said sections (225) decrease from the section (225) in the vicinity of said inner end (221) to the section (225) at the outer end (222).

11. Packaging according to claim 10, wherein said second pitch (d11) between first and second sections (225) in the vicinity of the inner end (221) is smaller than said first constant pitch (d10) of said first section (225) in the vicinity of the inner end (221); and said second pitch (d11) between first and second sections (225) at the outer end (222) is greater than said first constant pitch (d13) of said first section (225) at the outer end (222).

12. Packaging according to any one of claims 1 to 11, wherein said support strip (9; 109; 209) is made from a longitudinal single sheet of cardboard.

13. Packaging according to any one of claims 1 to 12, wherein said container (10; 110; 210) is a syringe barrel or an ampoule vial having a cylindrical shaped body, which is made from glass or plastic material.

14. Packaging according to any one of claims 1 to 13, wherein it further comprises a box (2; 202) having an internal space (8; 208) which is configured to receive said support strip (9; 109; 209), said support strip (9; 109; 209) being stored in said box (2; 202).

15. Packaging according to claim 14, wherein said box (2; 202) comprises lateral walls (3; 203) and a window (6; 206) provided in one of them, said window (6; 206) being configured to be through by said support strip (9; 109; 209) in order to dispense said containers (10; 110; 210).
